# EUROPEAN PATENT SPECIFICATION

(11) **EP 4 384 260 B1**
(45) Date of publication and mention of the grant of the patent: **01.10.2025**
(21) Application number: 22764662.7
(22) Date of filing: 08.08.2022
(51) Int. Cl.: A61N 1/05

(54) **BRAID-SUPPORTED HELICAL LEADS FOR IMPLANTABLE ELECTRODE LEADS**
GEFLOCHTENE SPIRALFÖRMIGE LEITUNGEN FÜR IMPLANTIERBARE ELEKTRODENLEITUNGEN
FILS HÉLICOÏDAUX SUPPORTÉS PAR UNE TRESSE POUR LES FILS D'ÉLECTRODES IMPLANTABLES

(30) Priority: 10.08.2021 DE 202021104261 U
(43) Date of publication of application: 19.06.2024
(73) Proprietor: BIOTRONIK SE & Co. KG, 12359 Berlin (DE)
(72) Inventor: RUMP, Jens, 12049 Berlin (DE); RICHTER, Jan Helge, 12355 BERLIN (DE); STEGLICH, Carsten, 15344 Strausberg (DE)
(74) Representative: Biotronik Corporate Services SE
(86) International application number: PCT/EP2022/072204
(87) International publication number: WO 2023/016973

(56) References cited:
- EP-A1- 1 935 449
- EP-A1- 2 564 892
- WO-A1-2020/260342
- US-B2- 9 517 335

## Description

The present invention relates to an implantable electrode lead.

Currently, two different types of leads to electric poles are mainly used in implantable electrodes, namely helical leads based on wire-shaped electrical conductors and comparatively straight leads based on rope-shaped electrical conductors.

Due to their plastic deformability, wires can be wound into helical leads relatively easily; however, precisely because of their plastic deformability, they are limited to the smallest possible pitches, as otherwise the fatigue strength suffers. Rope-shaped leads, on the other hand, are only slightly plastically deformable and therefore exhibit increased fatigue strength, but for this reason cannot be wound into coils. However, it is also possible to form rope-shaped leads into coils by supporting them in a dimensionally stable manner with additional electrical leads that are guided in a direction of rotation opposite to that of the rope-shaped leads.

Especially for electrode leads used in the therapy of tachycardia, which necessitates the delivery of high-voltage pulses for shock therapy, a minimum lead spacing is important and should not be undercut to avoid flashover voltages between leads. However, ensuring this minimum spacing with lead wires arranged in a cross pattern is proving to be extremely challenging.

WO 2020/260342 A1 discloses an implantable electrode lead, e.g., for MRI examination of patient, having conductors connected to one another to form braid with first conductors and second conductors helically wound in opposite rotation directions. US 9,517,335 B2 discloses an implantable electrical line including a lead, wherein the lead includes at least one helically wound electrical conductor, which is surrounded by a fiber braid, which is formed by at least two fibers or fiber bundles, and wherein the at least two fibers or fiber bundles are interwoven and wound around the electrical conductor in opposite winding directions.

Based on the above, the problem to be solved by the present invention is to provide an improved implantable electrode lead with helical leads.

This task is solved by an implantable electrode lead having the features of claim 1. Advantageous embodiments of the invention are stated in the corresponding dependent claims and are described below.

In the present invention, an implantable lead comprises:
- at least one electrode pole,
- at least one electrical conductor connected to the at least one electrode pole in an electrically conducting fashion, and
- at least one longitudinally extended electrical insulator,
wherein according to the invention,
the at least one electrical conductor is helically wound in a first direction of rotation about a longitudinal axis of the electrode lead, and wherein the at least one electrical insulator is helically wound in a second direction of rotation about the longitudinal axis opposite to the first direction of rotation, and
wherein theimplantable electrode lead is configured to be screwed into human or animal tissue, wherein a braid comprising the helically wound at least one electrical conductor and the helically wound at least one electrical insulator is configured to transfer torque upon screwing the implantable electrode lead into said tissue.

Particularly, said direction of rotation corresponds to the kind of helix (left-handed or right-handed) of the electrical conductor and electrical insulator, respectively. Particularly, the first direction of rotation can mean that the at least one electrical conductor forms a left-handed helix. Since the at least one electrical insulator comprises the opposite direction of rotation it forms a right-handed helix. Likewise, if in turn the at least one electrical conductor forms a right-handed helix, at least one electrical insulator forms a left-handed helix. Thus, opposite direction of rotation means opposite chirality (left-handed helix versus right-handed helix). Thereby the opposite direction of rotation is understood with respect to the longitudinal axis of the implantable electrode from the proximal to the distal end.

The structure, particularly braid, formed by said electrical conductor(s) and insulator(s) has the particular advantage of allowing to transfer torque while having a robust and flexible electrode lead at the same time.

In other words, the invention circumvents the problem described in the introduction by designing the braid-like structure in such a way that the electrically conductive leads are each helically shaped in only one direction and are supported in their helical form exclusively by insulators wound in opposite directions thereto.

The minimum distance between the electrical conductors can thus be achieved in a simplified manner by running insulators in parallel between the electrical conductors without allowing crossing points between the electrical conductors. In other words, electrical conductors run in parallel and do not cross each other. Electrical conductors are only crossed by insulators. Thereby the crossing of electrical conductors is avoided and a braid is established at the same time.

In principle, this concept is not limited to electrode leads for tachycardia treatment, but can also be applied to other electrode leads or catheters, particularly where cross-arranged conductors may be undesirable, e.g., to avoid subclavian crush syndrome, and structures allowing to transfer torque are desired.

In particular, the described method, with the possibility to scale the number of leads, can be used for catheters in electrophysiology, where many electrode leads are needed in a very small space. Conventional lead concepts with single wires quickly reach their limits here, if high-resolution mapping and differentiated temperature monitoring are to take place simultaneously during ablation at many positions, e.g. to realize linear lesions in a one-shot procedure.

The invention is defined in claim 1. Further aspects and preferred embodiments are defined in the appended claims.

In the following, embodiments and further features and advantages of the invention at hand will be described with reference to the Figures, wherein
- Fig. 1: shows a section of an embodiment of an implantable electrode lead according to the invention,
- Fig. 2: shows the section shown in Fig. 1, with an outer insulating tube and the electrode poles not shown, so that underlying electrical conductors or insulators are visible.
- Fig. 3: shows a section of a further embodiment of an implantable electrode lead according to the invention, wherein an outer electrically insulating layer covering the conductors and insulators is not shown,
- Fig. 4: shows a cross section of an electrical conductor in form of a rope of an embodiment of an implantable electrode lead according to the invention,
- Fig. 5: shows a cross section of an electrical conductor in form of a rope of an embodiment of an implantable electrode lead according to the invention, and
- Fig. 6: shows a cross section of an electrical conductor in form of a rope of an embodiment of an implantable electrode lead according to the invention.

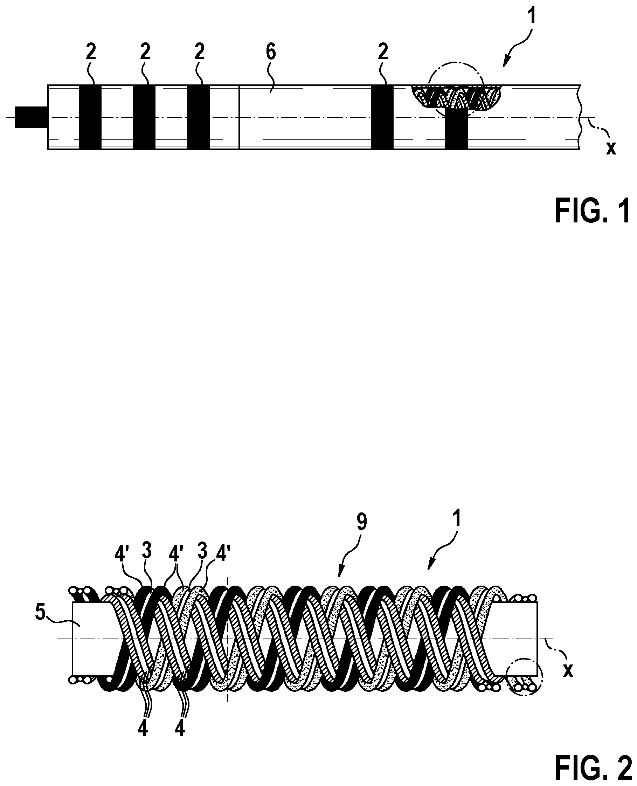

Fig. 1 shows in connection with Fig. 2 an embodiment of an implantable electrode lead 1, e.g. for the therapy of tachycardia, according to the present invention, the lead 1 comprising at least one electrode pole 2, at least one electrical conductor 3, which is electrically conductively connected to the at least one electrode pole 2, and comprising at least one longitudinally extended electrical insulator 4. According to the invention, the at least one electrical conductor 3 is helically wound in a first direction of rotation about a longitudinal axis x of the electrode lead 1, and that the at least one electrical insulator 4 is helically wound in a second direction of rotation opposite to the first direction of rotation about the longitudinal axis x.

The implantable electrode lead 1 may further comprise an insulating tube 5, e.g. made of silicone, having an inner diameter of e.g. 0.83 mm and an outer diameter of e.g. 1.13 mm. In the inner lumen of the hose 5, a coiled lead of wire may be arranged (not shown), which may be connected to a helical fixation at the distal end. The at least one conductor 3 and the at least one insulator 4 are arranged on the hose 5.

Preferably, multiple conductors 3 and insulators 4 are provided, as will be explained below with reference to Figures 1 and 2.

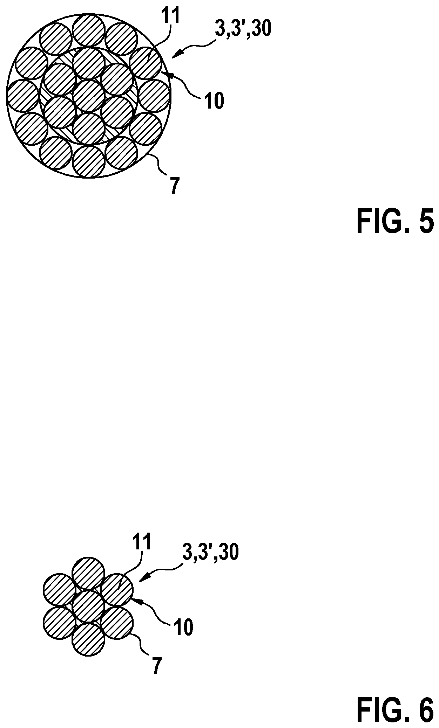

Preferably, at least two electrical conductors 3, each in the form of a rope-shaped insulated electrical lead with a diameter of, for example, 0.36 mm, are wound in parallel over the insulating tube 5 with two thread-shaped insulators 4' of, for example, polyurethane (PU) with diameters of, for example, 0.15 mm, or at least 0.15 mm, the two electrical conductors 3 being braided in parallel in one direction of rotation, at least one PU thread 4' in each case being located between the conductors 3 and at least 4 PU threads being braided in the opposite direction of rotation, for example (in Fig. 2, 6 PU threads or insulators 4 are laid in the opposite direction of rotation as an example).

The electrical conductors 3 are, for example, in one case, a high-impedance conductor 3 (e.g. MP35N), which may be in electrical connection in particular with a ring electrode 2 of the electrode line 1, which may be used to detect an electrical activity, and a low-impedance conductor 3, e.g. in the form of a DFT rope with a silver core, e.g. with electrical connection to a shock coil (not shown).

An electrically insulating layer 6, particularly an insulating hose 6 (e.g. silicone or PU) with an inside diameter of e.g. at least 2.15 mm and with an outside diameter of e.g. 2.5 mm is preferably mounted over the braiding of the conductors 3 and insulators 4, 4'. In addition, a PU hose can be mounted over the silicone hose.

For other types of tachycardia electrodes, e.g. electrodes with two shock coils or two additional sensing electrodes in the atrium (DX electrodes), the number of electrical conductors 3 can be increased accordingly.

To simplify contacting of the braided electrical conductors 3 with the outer electrode poles 2 or shock coils, the electrical conductors 3 can be provided with appropriate contact surfaces before the braiding process, e.g. in the form of electrically conductive tabs, which can be welded directly to the electrode poles 2. Subsequent stripping would be eliminated accordingly.

If the diameter of the electrically conductive lead wires 3 is to be reduced without increasing the electrical resistances to the same extent, two or more thinner rope-shaped lead wires can be braided in parallel with the same direction of rotation instead of one rope-shaped lead wire and connected together to an electrode pole 2.

Instead of using a filamentary insulating fiber 4 as part of the braid, insulating tubing can be used instead, which increases the overall diameter of the electrode less but at the same time ensures increased spacing between the electrical conductors 3 due to an elliptical deformation.

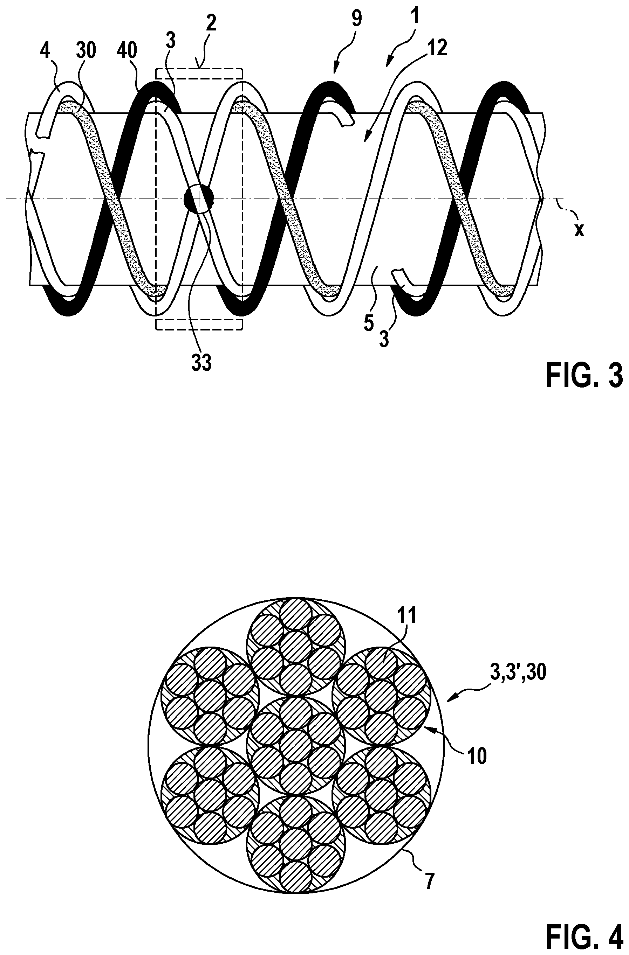

Fig. 3 shows a further embodiment of an implantable lead 1 according to the present invention, comprising a braid 9 of at least two electrical conductors 3, 30 wound in parallel in a helical fashion in a first direction of rotation (e.g. on a an electrically insulating carrier, e.g. tube 5), wherein at least two electrical insulators 4, 40 are helically wound in an opposite second direction of rotation (also in parallel). In other words, the conductors 3, 30 and insulators 4, 40 have opposite chiralities.

Preferably, the conductors 3, 30 used in the braid 9 running in parallel are ropes, which can be formed according to one of the embodiments shown in Figs. 4 to 6. Particularly, electrically conductive ropes are used for electrode leads especially when low resistances are required. This is particularly the case with tachycardia electrodes, where very high currents have to be transmitted. A tachycardia electrode has a resistance that is only between 1 and 10 ohms. If it were greater, the electrode would heat up during the shock. In addition, the voltage drop in the line would then be so large that the devices would have to provide significantly higher voltages.

For long-term implantation, copper as a particularly low-resistance metal is ruled out for reasons of biocompatibility. Instead, silver is preferably used as wire material, but because of its mechanical weaknesses, it is preferably supported by the very stable and corrosion-resistant MP35N (35wt% nickel; 35wt% cobalt; 20wt% chromium; and 10wt% molybdenum). A mechanically stable wire made of MP35N can contain a core of silver, which lowers the conductivity drastically. The wire can be wound into either a helix for mechanical loading in an electrode. A straight wire would break quickly in an electrode. Coiling has the disadvantage that the lead length and thus the resistance is relatively high. Alternatively, a wire 11 can be drawn very thin and many wires 11 combined into a rope. Such a rope is mechanically extremely stable against bending load changes (in some cases even more stable than a helix), it has an extremely low resistance because it can connect the conductors by the shortest path.

According to the embodiment shown in Fig. 4, ropes 3, 3', 30 that are preferably used (e.g. in the embodiments of Figs 1 to 3) are 7x7 ropes, i.e. seven wires 11 that may be formed out of the above-described materials, are twisted into a strand 10. Seven such strands 10 are in turn twisted into a rope 3, 3', 30.

Somewhat simpler are the 1x19 ropes as shown in Fig. 5. Here, a rope 3, 3', 30 can be formed out of 19 strands 10 each comprising a single wire 11. Such ropes may also be used for tachycardia electrodes and neuroelectrodes.

Finally, Fig. 6 shows an embodiment of a rope 3, 3', 30 suitable for thin neuroelectrodes, wherein here the rope comprises seven strands, each strand 10 formed from a single wire 11.

According to an embodiment, these ropes 3, 3', 30 are either made of solid MP35N wires - then they have a relatively high resistance, which is sufficient for sensing and stimulation, or they contain wires with a silver core, and are then very low resistance. The silver content can vary and determines the compromise between low resistance and mechanical stability. Wires with a silver core are used especially for tachy probes and neuroprobes. For neuroprobes, the silver core is less important because of high energies than because these ropes are very thin and still need to be conductive.

Preferably, the respective rope 3, 3', 30 shown in Figs. 4 to 6 is preferably electrically insulated, i.e. surrounded by an electrically insulating cladding 7. This cladding 7 can be a coating. A corresponding material of the cladding/coating 7 is typically made of ETFE (ethylene tetrafluoroethylene) or PFA (perfluoroalkoxy polymers). Other claddings/coatings are also conceivable: Polyurethane (PU), polyester urethanes (PEU), polyether urethanes (PEEU), polycarbonate urethanes (PCU), silicone-based polycarbonate urethanes (PCU) bond better with silicone during extrusion, polycarbonate polyurea urethanes (PCHU), polydimethylsiloxane urethanes (PSU), polyisobutylene urethanes (PIU), polyisobutylene-based copolymers (PIC), polyether block amides (PEBA, e.g. PEBAX), polyimides (PI), fluorinated hydrocarbons, ethylene-tetrafluoroethylene copolymer (ETFE), polytetrafluoroethylene, polysulfone (PSU), polyethylene (PE), polypropylene (PP), polyamides (PA), silicone, polyimides (PI), fluorinated hydrocarbons, polytetrafluoroethylene (PTFE), tetrafluoroethylene (TFE), perfluoro(ethylene-propylene) (FEP), PEEK.

Due to said electrical insulation by means of cladding 7 electrolyte bridges between conductors 3, 30 can be prevented from forming when body fluid enters.

The concept according to the invention is to be used especially with tachycardia electrodes, which, unlike conventional tachycardia electrodes- are intended to be screwed into the septum to sense and stimulate close to the LBB (left bundle branch). Typically, electrodes that are actively fixed in the tissue use a helix for fixation. This helix has a length of e.g. 2mm. A longer helix is also conceivable. However, if the LBB has to be reached at a depth of e.g. 8mm, the helix would have to be partially insulated in order not to unnecessarily dissipate (waste) current over the full length during stimulation. It seems easier to leave the helix short and let the electrode body follow the helix into the septum until the helix reaches the LBB. A classic helical electrode is fixed by transferring the rotation at the connector pin to the helix via an inner conductor helix, which then extends out of the helix body and anchors itself in the tissue. This technique is useful for classical electrode applications because it prevents potential perforation of the cardiac tissue. The inner conductor has a limited torque that allows the helix to be very easily screwed into the tissue until the electrode head (housing) is in contact with the tissue. For a normal RA or RV implantation, this is useful to protect against perforation, because if the free heart wall is perforated, the electrode tip will penetrate into the pericardium, where it will cause tamponade on the one hand and will not be able to stimulate on the other hand.

In the present application, however, the septum is to be penetrated deliberately by the electrode so that preferably the deep LBB is reached. Torque transmission through the inner conductor (e.g. arranged in tube 5) is only moderately suitable because very many turns would be required to allow the electrode to penetrate. Therefore, the screw that can be screwed out is often omitted and a fixed screw is used, which is rotated by holding on to the electrode lead 1 and turning the electrode lead 1. A higher torque can be applied due to the braid 9 than with an inner helix. However, it is also conceivable to unscrew the helix with the connector pin, preload the inner conductor by overtightening the connector pin, and then implant the electrode lead 1 by turning it.

Tachycardia electrodes are popularly constructed with a multilumen tubing as the electrode body. Even the multilumen tubing cannot transmit much torque because the soft tubing material (silicone or polyurethane) twists easily.

However, the braid 9 of conductors 3, 30 and insulators 4, 40 arranged in opposite directions transmits the torque very well. The disadvantage of a braid is that the filaments may cross each other. If the filaments consist of feed conductors, there is a risk that the insulation will be compressed at the crossing points. Even if the conductors do not touch, there is a risk of breakdown when the shock is applied, especially in tachycardia applications.

In the present embodiment, therefore, the conducting ropes 3, 30 are guided in one direction of rotation (chirality), while the crossing insulators 4, 40 (e.g. plastic filaments) consist of insulating materials and are guided in the opposite direction of rotation (opposite chirality). In this way, the conductors 3, 30 are fixed in position but run completely parallel in the braid 9 without overlapping (c.f. e.g. Fig. 3). In further embodiments, the conductors 3, 30 running in parallel may also alternate with insulators in addition. However, one of the advantageous features of this embodiment is that conductors 3, 30 do not cross in the braid 9.

Furthermore, an electrically insulating layer 6 is preferably applied to the braid 9 (not shown in Fig. 3 so that conductors 3, 30 / insulators 4, 40 are visible) to prevent e.g. direct exposure to blood. A raw braid structure 9 would grow very much into the heart and vessels. This would make explantation of the electrodes very difficult. A tubing (e.g., polyurethane, silicone, or a copolymer) 6 can be used as a covering 6. In one embodiment, this tubing 6 is connected very tightly to the braid 9. This has the advantage that the electrode lead 1 is particularly easy to grip and turn by hand. The torque applied by the fingers is transmitted directly to the braid 9. This condition can be e.g. achieved by over-extruding the insulating layer 6 made of polyurethane or a copolymer or of silicone.

According to a further embodiment, a reflow process can be utilized. In this process, a tube 6 made of a thermoplastic is pushed over the braid 9 and shrunk in a heat process, melting it into the braid structure 9. The electrical conductors 3, 30 are routed continuously in the braid, which means that the conductor 3 of a ring electrode 2 that is connected at connection point 33 to the ring electrode 2 (cf. Fig. 3), for example, also runs under a shock coil (a tachycardia electrode arranged in the form of a coil configured for delivering high-voltage energy). This could lead to a short-circuit in the event of a run.

According to an embodiment, the conductor 3 can be interrupted, e.g. for one mesh size as shown in Fig. 3 showing a corresponding gap 12, so that this conductor 3 is electrically inactive below the shock coil, but the actual braiding remains connected and its mechanical properties are almost unaffected.

Preferably, plastic threads can be used as insulators 4, 40; these form the braid 9 in the opposite direction of rotation (chirality) to the electrical conductors 3, 30. At the crossing points, the threads lay very flat due to their thread structure and the thread tension. The plastic threads used as insulators 4, 40 are configured to be radially elastic.

This has the considerable advantage that the inner and outer diameters can be kept small, since only the cross-section of the electrical conductor 3, 30 (e.g. the diameter of the cable, but not of the filament) is included in the intended wall thickness of the braid 9.

The present invention simplifies the production of an implantable electrode lead, can be advantageously applied to electrode leads of various types, and avoids crossing points of conductors, which increases safety against high-voltage flashovers. Furthermore, the number of leads can be increased. Further, at the same time, the braid formed from the crossing electrical conductors and electrical insulators allows to transfer torque in an advantageous fashion.

## Claims

1. An implantable electrode lead (1), comprising:
- at least one electrode pole (2),
- at least one electrical conductor (3, 30), which is electrically conductively connected to the at least one electrode pole (2), and
- at least one longitudinally extended electrical insulator (4, 40)
wherein the at least one electrical conductor (3, 30) is helically wound in a first direction of rotation about a longitudinal axis (x) of the electrode lead (1), and in that the at least one electrical insulator (4, 40) is helically wound in a second direction of rotation, opposite to the first direction of rotation, about the longitudinal axis (x);
and **characterized in that** the implantable electrode lead (1) is configured to be screwed into human or animal tissue, wherein a braid (9) comprising the helically wound at least one electrical conductor (3, 30) and the helically wound at least one electrical insulator (4, 40) is configured to transfer torque upon screwing the implantable electrode lead (1) into said tissue.

2. The implantable electrode lead according to claim 1, wherein the implantable electrode lead comprises a further electrical conductor (30) helically wound in the first direction of rotation about the longitudinal axis (x).

3. The implantable electrode lead according to claim 2, wherein the further electrical conductor (30) is electrically conductively connected to a further electrode pole (2) of the implantable electrode lead (1).

4. The implantable electrode lead according to one of the preceding claims, wherein the implantable electrode lead comprises a further electrical insulator (40) helically wound in the second direction of rotation about the longitudinal axis.

5. The implantable electrode lead according one of the preceding claims, wherein the respective electrode pole (2) is a ring electrode.

6. The implantable electrode lead, wherein the respective electrical conductor (3, 3', 30) is a rope comprising a plurality of strands (10).

7. The implantable electrode lead according to claim 6, wherein the respective strand (10) comprises at least one wire (11).

8. The implantable electrode lead according to claim 6 or 7, wherein the respective rope comprises seven strands (10), the respective strand (10) comprising seven twisted wires (11).

9. The implantable electrode lead according to claim 6 or 7, wherein the respective rope comprises 19 strands (10), the respective strand (10) comprising one wire (11).

10. The implantable electrode lead according to claim 6 or 7, wherein the respective rope comprises 7 strands (10), the respective strand (10) comprising one wire (11).

11. The implantable electrode lead according to one of the claims 7 to 10, wherein the respective wire comprises or is formed out a material selected from the list comprised of: Silver, an alloy comprising nickel and cobalt, MP35N.

12. The implantable electrode lead according to one of the claims 7 to 11, wherein the respective wire comprises a core comprising a first metal and a sheath surrounding the core, the sheath being formed out of a different second metal.

13. The implantable electrode lead according to one of the claims 6 to 12, wherein the respective rope is covered with an electrically insulating cladding (7).

14. The implantable electrode lead according to one of the preceding claims, wherein the implantable electrode lead comprises an electrically insulating layer (6) covering the at least one electrical conductor (3, 3', 30) and the at least one electrical insulator (4, 40).

## Patentansprüche

1. Eine implantierbare Elektrodenleitung (1), umfassend:
- mindestens einen Elektrodenpol (2),
- mindestens einen elektrischen Leiter (3, 30), der mit dem mindestens einen Elektrodenpol (2) elektrisch leitend verbunden ist, und
- mindestens einen in Längsrichtung verlaufenden elektrischen Isolator (4, 40)
**wobei**
der mindestens eine elektrische Leiter (3, 30) in einer ersten Drehrichtung um eine Längsachse (x) der Elektrodenleitung (1) spiralförmig gewickelt ist, und dass der mindestens eine elektrische Isolator (4, 40) in einer zweiten, der ersten Drehrichtung entgegengesetzten Drehrichtung um die Längsachse (x) spiralförmig gewickelt ist;
und **dadurch gekennzeichnet, dass** die implantierbare Elektrodenleitung (1) so konfiguriert ist, dass sie in menschliches oder tierisches Gewebe eingeschraubt werden kann, wobei ein Geflecht (9), das den spiralförmig gewickelten mindestens einen elektrischen Leiter (3, 30) und den spiralförmig gewickelten mindestens einen elektrischen Isolator (4, 40) umfasst, so konfiguriert ist, dass es beim Einschrauben der implantierbaren Elektrodenleitung (1) in das Gewebe ein Drehmoment überträgt.

2. Die implantierbare Elektrodenleitung nach Anspruch 1, wobei die implantierbare Elektrodenleitung einen weiteren elektrischen Leiter (30) aufweist, der in der ersten Drehrichtung spiralförmig um die Längsachse (x) gewickelt ist.

3. Die implantierbare Elektrodenleitung nach Anspruch 2, wobei der weitere elektrische Leiter (30) elektrisch leitend mit einem weiteren Elektrodenpol (2) der implantierbaren Elektrodenleitung (1) verbunden ist.

4. Die implantierbare Elektrodenleitung nach einem der vorhergehenden Ansprüche, wobei die implantierbare Elektrodenleitung einen weiteren elektrischen Isolator (40) aufweist, der in der zweiten Drehrichtung spiralförmig um die Längsachse gewickelt ist.

5. Die implantierbare Elektrodenleitung nach einem der vorhergehenden Ansprüche, wobei der jeweilige Elektrodenpol (2) eine Ringelektrode ist.

6. Die implantierbare Elektrodenleitung, wobei der jeweilige elektrische Leiter (3, 3', 30) ein Seil mit mehreren Litzen (10) ist.

7. Die implantierbare Elektrodenleitung nach Anspruch 6, wobei die jeweilige Litze (10) mindestens einen Draht (11) umfasst.

8. Die implantierbare Elektrodenleitung nach Anspruch 6 oder 7, wobei das jeweilige Seil sieben Litzen (10) umfasst, wobei die jeweilige Litze (10) sieben verdrillte Drähte (11) umfasst.

9. Die implantierbare Elektrodenleitung nach Anspruch 6 oder 7, wobei das jeweilige Seil 19 Litzen (10) umfasst, wobei die jeweilige Litze (10) einen Draht (11) umfasst.

10. Die implantierbare Elektrodenleitung nach Anspruch 6 oder 7, wobei das jeweilige Seil 7 Litzen (10) umfasst, wobei die jeweilige Litze (10) einen Draht (11) umfasst.

11. Die implantierbare Elektrodenleitung nach einem der Ansprüche 7 bis 10, wobei der jeweilige Draht ein Material umfasst oder daraus geformt ist, das aus der Liste ausgewählt ist, die umfasst: Silber, eine Legierung aus Nickel und Kobalt, MP35N.

12. Die implantierbare Elektrodenleitung nach einem der Ansprüche 7 bis 11, wobei der jeweilige Draht einen Kern aus einem ersten Metall und einen den Kern umgebenden Mantel aus einem anderen zweiten Metall aufweist.

13. Die implantierbare Elektrodenleitung nach einem der Ansprüche 6 bis 12, wobei das jeweilige Seil mit einer elektrisch isolierenden Verkleidung (7) überzogen ist.

14. Die implantierbare Elektrodenleitung nach einem der vorhergehenden Ansprüche, wobei die implantierbare Elektrodenleitung eine elektrisch isolierende Schicht (6) aufweist, die den mindestens einen elektrischen Leiter (3, 3', 30) und den mindestens einen elektrischen Isolator (4, 40) verhüllt.

## Revendications

1. Câble d'électrode implantable (1), comprenant :
- au moins un pôle d'électrode (2),
- au moins un conducteur électrique (3, 30), qui est électriquement connecté de manière conductrice à l'au moins au pôle d'électrode (2), et
- au moins un isolant électrique à extension longitudinale (4, 40)
**dans lequel**
l'au moins un conducteur électrique (3, 30) est enroulé en hélice dans un premier sens de rotation autour d'un axe longitudinal (x) du câble d'électrode (1), et en ce que l'au moins un isolant électrique (4, 40) est enroulé en hélice dans un second sens de rotation, opposé au premier sens de rotation, autour de l'axe longitudinal (x) ;
et **caractérisé en ce que** le câble d'électrode implantable (1) est conçu pour être vissé dans un tissu humain ou animal, dans lequel une tresse (9) comprenant l'au moins un conducteur électrique (3, 30) enroulé en hélice et l'au moins un isolant électrique (4, 40) enroulé en hélice est conçu pour transférer un couple lors du vissage du câble d'électrode implantable (1) dans ledit tissu.

2. Câble d'électrode implantable selon la revendication 1, dans lequel le câble d'électrode implantable comprend un conducteur électrique (30) supplémentaire enroulé en hélice dans le premier sens de rotation autour de l'axe longitudinal (x).

3. Câble d'électrode implantable selon la revendication 2, dans lequel le conducteur électrique (30) supplémentaire est électriquement connecté de manière conductrice à un pôle d'électrode (2) supplémentaire du câble d'électrode implantable (1).

4. Câble d'électrode implantable selon l'une des revendications précédentes, dans lequel le câble d'électrode implantable comprend un isolant électrique (40) supplémentaire enroulé en hélice dans le second sens de rotation autour de l'axe longitudinal.

5. Câble d'électrode implantable selon l'une des revendications précédentes, dans lequel le pôle d'électrode (2) respectif est une électrode annulaire.

6. Câble d'électrode implantable, dans lequel le conducteur électrique (3, 3', 30) respectif est une corde comprenant une pluralité de brins (10).

7. Câble d'électrode implantable selon la revendication 6, dans lequel le brin (10) respectif comprend au moins un fil (11).

8. Câble d'électrode implantable selon la revendication 6 ou 7, dans lequel la corde respective comprend sept brins (10), le brin (10) respectif comprenant sept fils (11) torsadés.

9. Câble d'électrode implantable selon la revendication 6 ou 7, dans lequel la corde respective comprend 19 brins (10), le brin (10) respectif comprenant un fil (11).

10. Câble d'électrode implantable selon la revendication 6 ou 7, dans lequel la corde respective comprend 7 brins (10), le brin (10) respectif comprenant un fil (11).

11. Câble d'électrode implantable selon l'une des revendications 7 à 10, dans lequel le fil respectif comprend ou est formé d'un matériau choisi dans la liste constituée par : l'argent, un alliage comprenant du nickel et du cobalt, MP35N.

12. Câble d'électrode implantable selon l'une des revendications 7 à 11, dans lequel le fil respectif comprend une âme comprenant un premier métal et une gaine entourant l'âme, la gaine étant formée d'un second métal différent.

13. Câble d'électrode implantable selon l'une des revendications 6 à 12, dans lequel la corde respective est recouverte d'un revêtement électriquement isolant (7).

14. Câble d'électrode implantable selon l'une des revendications précédentes, dans lequel le câble d'électrode implantable comprend une couche électriquement isolante (6) recouvrant l'au moins un conducteur électrique (3, 3', 30) et l'au moins un isolant électrique (4, 40).
